Europäisches Patentamt

European Patent Office

Office européen des brevets

(11) Publication number: **0 428 474 A2**

# EUROPEAN PATENT APPLICATION

(21) Application number: **90630192.4**

(22) Date of filing: **09.11.90**

(51) Int. Cl.5: **A61N 2/08**

(30) Priority: **13.11.89 US 434458**

(43) Date of publication of application:
**22.05.91 Bulletin 91/21**

(84) Designated Contracting States:
**CH DE FR GB IT LI SE**

(71) Applicant: **HOLCOMB MEDICAL CORPORATION**
**Universal Pain Management Centre, Suite 1150, 2100 West End Avenue**
**Nashville, Tennessee 37203(US)**

(72) Inventor: **Holcomb, Robert R.**
**Post Office Box 779**
**Hamilton Alabama 35570(US)**

(74) Representative: **Meyers, Ernest et al**
**Office de Brevets FREYLINGER & ASSOCIES**
**B.P. 1 321, route d'Arlon**
**L-8001 Strassen(LU)**

(54) Therapeutic magnetic shoe and method of application.

(57) A therapeutic shoe and method for increasing blood circulation, reducing fatigue and reducing pain in a foot on which the shoe is worn is provided. The shoe has at least one magnetic treatment device mounted to abut the foot on which the shoe is worn. The magnetic treatment device includes a plurality of magnet bodies having two positive and two negative magnetic poles held in a fixed quadrilateral orientation.

*FIG. 1*

The present invention relates to magnetic devices for therapeutic application to the human body, and more particularly to a quadripolar static magnetic device adapted for utilization in the soles of shoes.

Magnetic fields have been applied to the human body for various therapeutic purposes. For example, magnetic plasters for improving circulation are disclosed in United States Patent Number 4,489,711, magnetic fields for stimulation of bone growth are disclosed in U.S. Patent No. 4,105,017, and magnetic stimulation of nerve cells has been accomplished with devices such as the Cadwell Magneto-Electric Stimulator (MES-10) manufactured by Cadwell Laboratories, Inc. of Kennewick, Washington.

Recent studies indicate that static magnetic fields may be therapeutically applied in order to alter the behavior of nerve tissue. In a study, Hong, Harmon & Yu: Static Magnetic Field Influence on Rat Tail Nerve Function, 76 Arch. Phys. Med. Rehabil. 746-49 (1986), a static magnetic field having a density greater than 0,5 Telsa was found to alter nerve function when applied for a duration of at least 30 seconds. The Hong study postulates that an electromagnetic field may relieve pain by selectively increasing the excitability of large nerve fibers which, according to the gate control theory, may block the gate for pain. This study also suggests that static magnetic fields alter nerve function by stabilizing nerve cell membranes and the permeability of the membranes to certain ions.

Pain sensations in the human body can be a result of improper nerve function, as when such pain is caused by inordinately excitable nerve cells or by nerve cells having leaky cell wall membranes. Pain sensations may also be caused by damaged nerve cells, as for example nerve cells suffering from post-operative scarring or physically impinged nerve cells commonly associated with degenerative disc disease. Even when nerves function properly, fatigue and pain sensations are initiated through nerve cells. Thus, relief from foot pain and fatigue should be obtainable by altering nerve cell function, as for example by stabilizing nerve cell wall membranes.

Unfortunately, many types of foot fatigue and pain cannot be successfully treated with conventional drug or physical therapies. Because foot fatigue and pain induced by standing, walking or running are often untreatable with conventional therapies, there is a need for alternative therapies that relieve these symptoms by altering nerve function and blood circulation in the feet. Accordingly, it is an object of the invention to provide a magnetic device and method for applying the device against the human foot so as to alter nerve behavior in a manner that reduces foot fatigue and pain. It is a further object of the invention to provide a magnetic device and a method for applying the device against the human foot so as to improve blood circulation.

Additional objects and advantages of the present invention will be set forth in part in the description that follows and, in part, will be obvious from the description or may be learned by practice of the invention. The objects and advantages of the invention may be realized and obtained by the appartus and method particularly pointed out in the appended claims.

## SUMMARY OF THE INVENTION

In accordance with the principles of the present invention, as embodied and as broadly described herein, a shoe sole with a therapeutic permanent magnet device embedded therein is provided. The permanent magnet device comprises a plurality of magnet bodies having at least two positive and two negative magnetic poles substantially in a single plane, the magnetic poles being oriented to define the four vertices of a quadrilateral shape, the two positive poles defining opposite diagonal vertices, and the two negative poles defining opposite diagonal vertices of the quadrilateral shape. Containment means are provided for holding the magnetic poles of the magnetic bodies in the quadrilateral orientation. The plurality of magnet bodies may comprise four substantially identical cylindrical magnetic bodies, each having at least one magnetically charged face. It is preferred that two negative magnetic poles on the magnetically charged faces of the four magnet bodies be in the quadrilateral orientation described above.

In accordance with the invention, at least one therapeutic permanent magnet device is embedded in a shoe. Preferably one permanent magnet device is embedded in the heel of the shoe's sole while a second device is embedded in the portion of the shoe's sole corresponding to the ball of the foot. The shoe into which the magnetio treatment devices are embedded may be of any type of footwear, as for example, running shoes, nurses shoes or work boots.

The accompanying drawings, which are incorporated in and constitute a part of this specification, illustrate presently preferred embodiments of the invention, and, together with the description, serve to explain the principles of the invention.

## BRIEF DESCRIPTION OF THE DRAWINGS

Fig. 1 is a side cross-sectional view of a shoe according to one embodiment of the invention;

Fig. 2 is a top cross-sectional view of the sole of the shoe of Fig. 1.

Fig. 3 is a prospective view of the magnetic treatment device embedded in the sole of the shoe of Fig. 1.

Fig. 4 is a plan view of the magnetic treatment device of Fig. 3.

Fig. 5 is a cross-sectional view taken along the line V-V of Fig. 3.

Fig. 6 is a front view of one of the magnetic bodies of the magnetic treatment device of Fig. 3.

Fig. 7 is a plan view of a second embodiment of the magnetic treatment device used in the invention.

Fig. 8 is a cross-sectional view taken along the line VIII-VIII of Fig. 7.

Fig. 9 is a top view of the magnetic treatment device of Fig. 4, with dots added to designate surface magnetic field measurement locations.

Fig. 10 is a graph of magnetic field measurements taken at the measurement locations designated in Fig. 9.

Fig. 11-17 are grids of magnetic field measurements taken at various distances from the surface of the magnetic treatment device of the present invention.

## DESCRIPTION OF THE PREFERRED EMBODIMENTS

Reference will now be made in detail to the presently preferred embodiments of the invention, examples of which are illustrated in the accompanying drawings. Throughout the drawings, like reference characters are used to designate like elements.

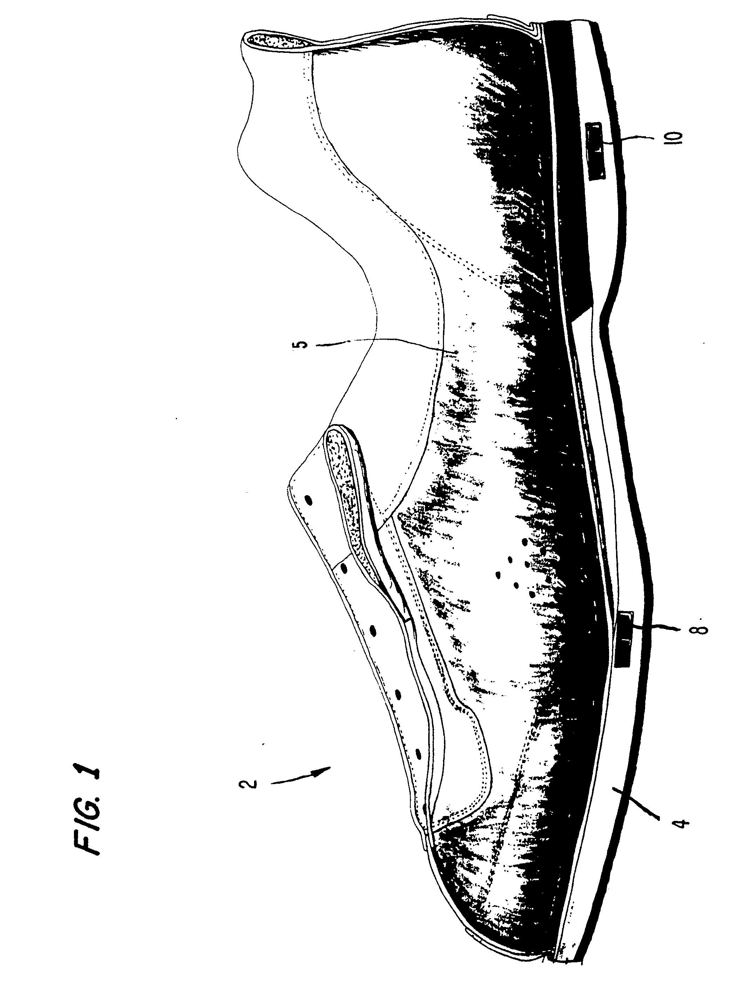

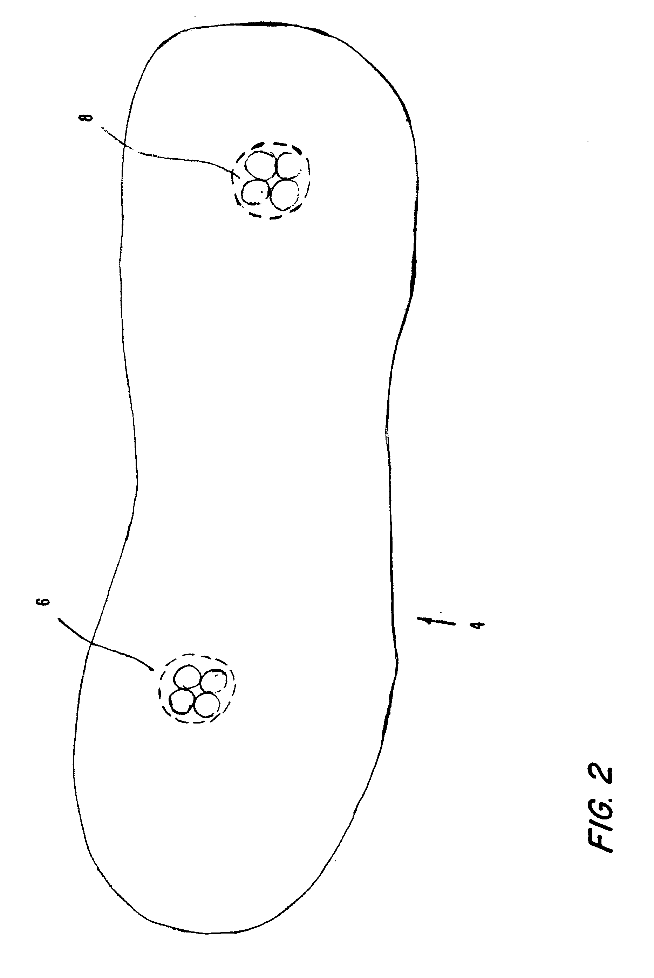

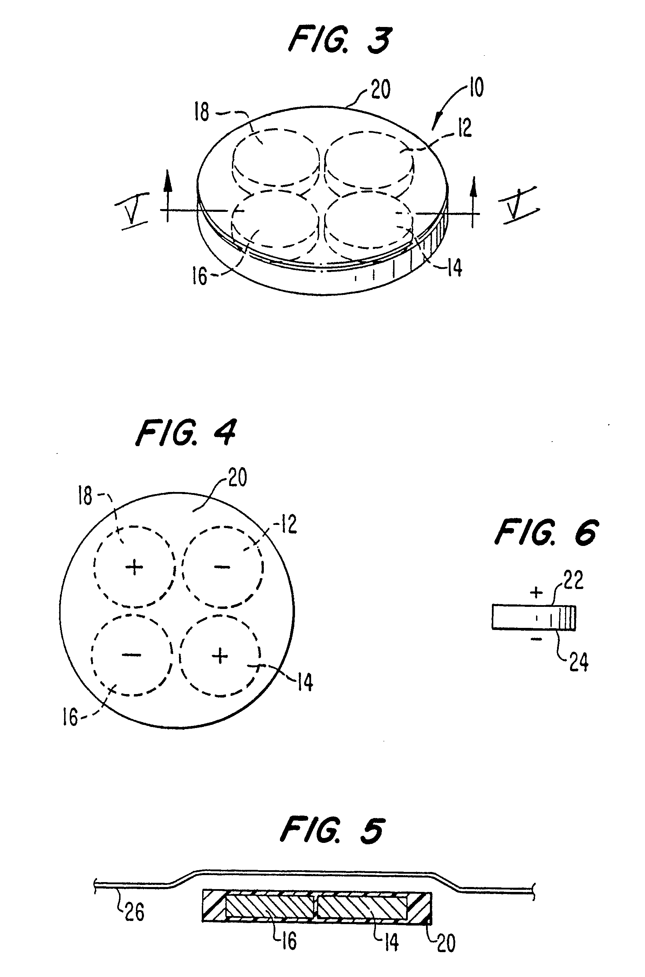

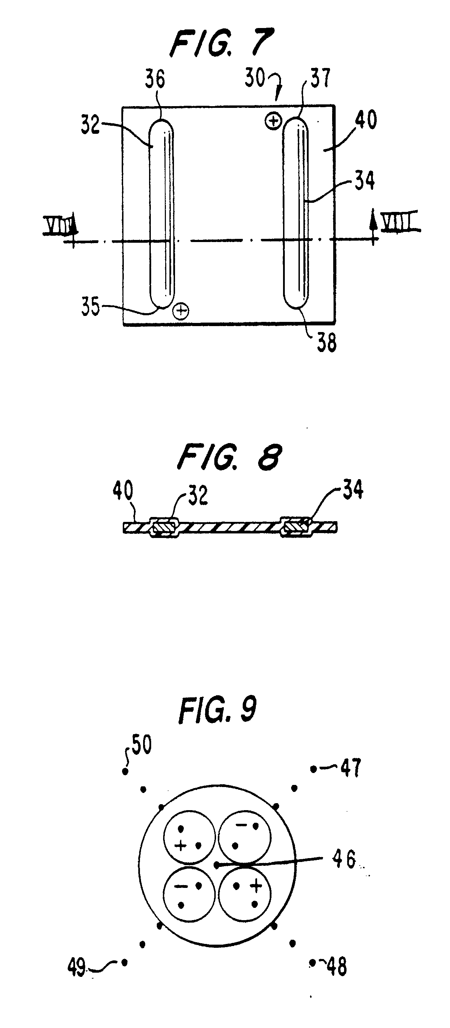

A side cross-sectional view of one preferred embodiment of the invention is shown in Fig. 1. A running shoe 2 has magnetic treatment devices 8 and 10 embedded in sole 4 of running shoe 2. In other embodiments of the invention, running shoe 2 could be any other type of footwear, as for example, a work boot or a nurse's shoe. As best shown in Fig. 2, magnetic treatment device 8 is embedded in the portion of sole 4 abutting the metatarsal heads (ball of the foot) of a foot in shoe 2. Magnetic treatment device 10 is embedded in the portion of sole 4 abutting the heel of a foot in shoe 2. It is anticipated that magnetic treatment devices could be otherwise positioned in shoe sole 4 to abut against other parts of a foot in shoe 2. It is further anticipated that magnetic treatment devices could be implanted in the upper portions 5 of shoe 2 to abut against side or top portions of a foot in shoe 2. Two embodiments of permanent magnet treatment devices 8 and 10 are shown in Figs. 3 and 7. In each embodiment of the invention, a plurality of magnet bodies are oriented and held in a containment body.

The invention embodiment shown in Fig. 3 includes a permanent magnet treatment device 10 having a plurality of magnet bodies 12, 14, 16 and 18 held in a housing 20. According to the invention, the plurality of magnet bodies have at least two positive and two negative magnetic poles substantially in a single plane, the magnetic poles being oriented to define four vertices of a quadrilateral shape, the two positive poles defining opposite diagonal verticies and the two negative poles defining opposite diagonal verticies of the quadrilateral shape.

As embodied herein, four permanent magnet bodies 12, 14, 16 and 18 are provided each having a generally cylindrical shape and two opposite faces. As shown in Fig. 6, one face 22 of each cylindrical body is positively charged, and the opposite face 24 of each cylindrical body is negatively charged. Preferably, a positive magnetic pole is located on face 22, while a negative magnetic pole is located on face 24. As shown is Figs. 3 and 4, magnetic bodies 12, 14, 16 and 18 are oriented to define the four vertices of a quadrilateral shape. The quadrilateral shape is preferably a square, as shown in Fig. 4, wherein the positively charged faces of magnetic bodies 14 and 18 and the negatively charged faces of magnetic bodies 12 and 16 are in a single plane. The two positive poles on faces 14 and 18 define opposite diagonal vertices of the square shape, while the two negative poles on the faces of magnetic bodies 12 and 16 define opposite diagonal vertices of the square shape. Each of the four magnetic poles is magnetically attracted by the two oppositely charged poles and is magnetically repelled by the like charged pole.

Preferably, each of the magnet cylinders comprises a neodymium magnet, but other magnetic bodies, such as samarium-cobalt magnets, ferrite magnets and various rare earth magnets, may also be advantageously utilized in the device. Each of the magnet bodies, preferably exhibits an energy product of at least 25 MG-Oe, and most preferable exhibits an energy product of 27 MG-Oe. Neodymium magnets manufactured by Delco Remy, a division of General Motors Corporation, exhibit the desired energy products and can be advantageously applied in the present invention. Preferably, the diameter of each magnet body is equal and is within the range of 6,35 mm to 12,7 mm. It is further preferred that each magnetic body have an equal thickness.

When cylindrical magnet bodies with opposite poles on opposite faces are utilized in the invention, opposite sides of magnetic device 10 will exhibit the same quadripolar magnetic charge distribution. Whichever side of magnetic treatment device 10 is positioned to face a foot in shoe 2, the therapeutic effect will be the same.

According to the invention, containment means may be provided for holding the magnetic poles of the magnetic bodies in the desired orientation. Containment body 20 may comprise a plastic or rubber body in which four magnetic bodies 12, 14, 16 and 18 are embedded. Containment body 20 may also comprise any thin woven sheath for holding magnetic bodies 12, 14, 16 and 18 in the desired quadripolar orientation. Alternatively, magnetic bodies 12, 14, 16 and 18 may be directly embedded in shoe sole 4 such that shoe sole 4 holds magnetic bodies 12, 14, 16 and 18 in the quadripolar orientation.

According to a second embodiment of the invention, as shown in Fig. 7, the therapeutic permanent magnet device of the invention may comprise two elongated permanent magnet bodies, 32 and 34, each having a positive pole and a negative pole. As shown in Fig. 8, elongated magnet bodies 32 and 34 are embedded in a containment body 40 comprised of plastic or rubber. Positive pole 35 and negative pole 36 of magnetic body 32 and positive pole 37 and negative pole 38 of magnetic body 34 are oriented to define the four vertices of a rectangular shape in which positive poles 35 and 37 define opposite diagonal vertices of the rectangular shape and negative poles 36 and 38 define opposite diagonal vertices of the rectangular shape. The embodiment of the invention shown in Figs. 7 and 8 is fixed in a shoe in the same manner as disclosed for the invention embodiment shown in Figs. 2-5.

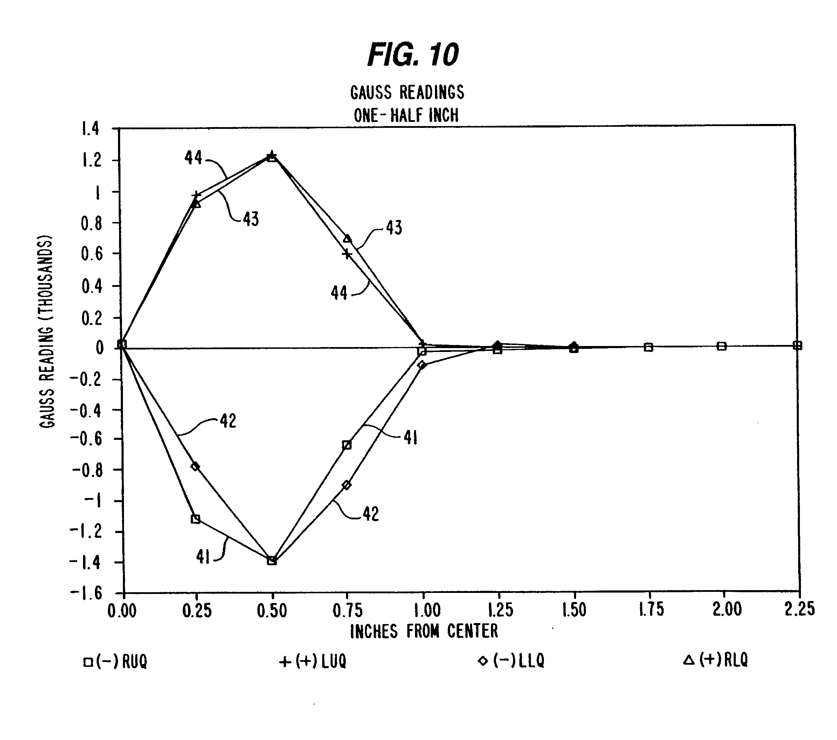

The magnetic field generated by a magnetic treatment device like that shown in Fig. 3 is quantified in the graphs and charts of Figs. 10-17. The measurements in Figs. 10-17 were made on a magnetic treatment device having the orientation shown in Fig. 3, in which each of the four magnetic bodies was a 12,7 mm diameter neodymium magnet, having an energy product of 27 MG-Oe. The magnetic field measurements were taken with a Gauss meter made by Applied Magnetic Laboratory, Inc., and are recorded in Gauss.

Fig. 10 is a graph of magnetic field measurements taken along the surface of a quadripolar magnetic treatment device like the one shown in Fig. 3 at measurement locations spaced 0.25 inch apart along diagonal lines as shown in Fig. 9. Graph line 41 in Fig. 10 corresponds to measurements taken along the diagonal 46-47 in Fig. 9; graph line 42 in Fig. 10 corresponds to measurements taken along the diagonal 46-49 in Fig. 9; graph line 43 corresponds to measurements taken along the diagonal 46-48 in Fig. 9; and graph line 44 corresponds to measurements taken along the diagonal 46-50 in Fig. 9. As shown in Fig. 10, magnetic field strength is negligible at the center of the magnetic treatment device, is greatest at one-half inch from the center of the magnetic treatment device in each of the four diagonal directions and decreases to almost nothing at 1.25 inches from the center of the magnetic treatment device. The quadripolar arrangement of the present invention provides a magnetic field coning effect, wherein the magnetic field is negligible at the center of the treatment device and is greatest at approximately one-half inch from the center of the treatment device.

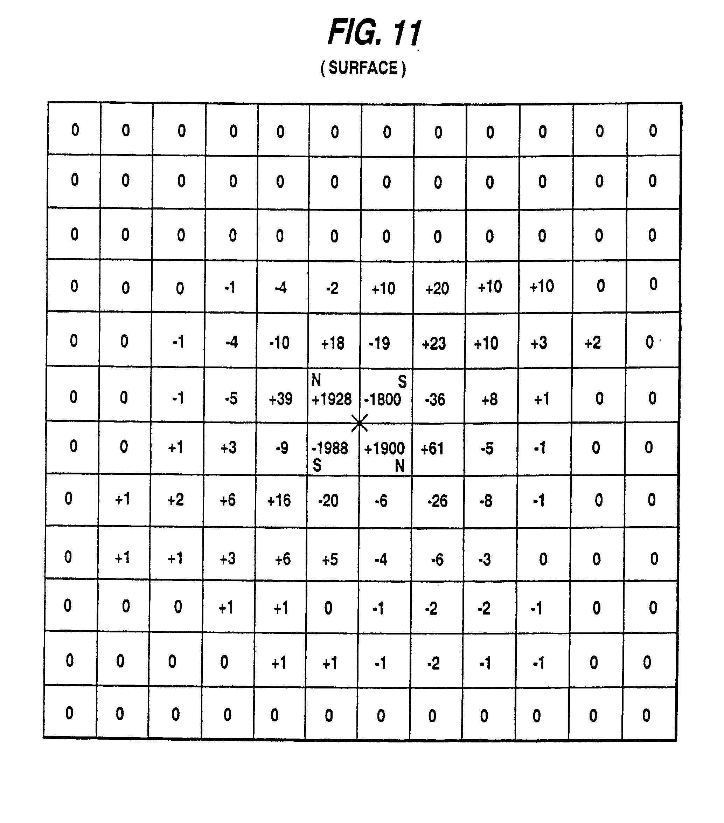

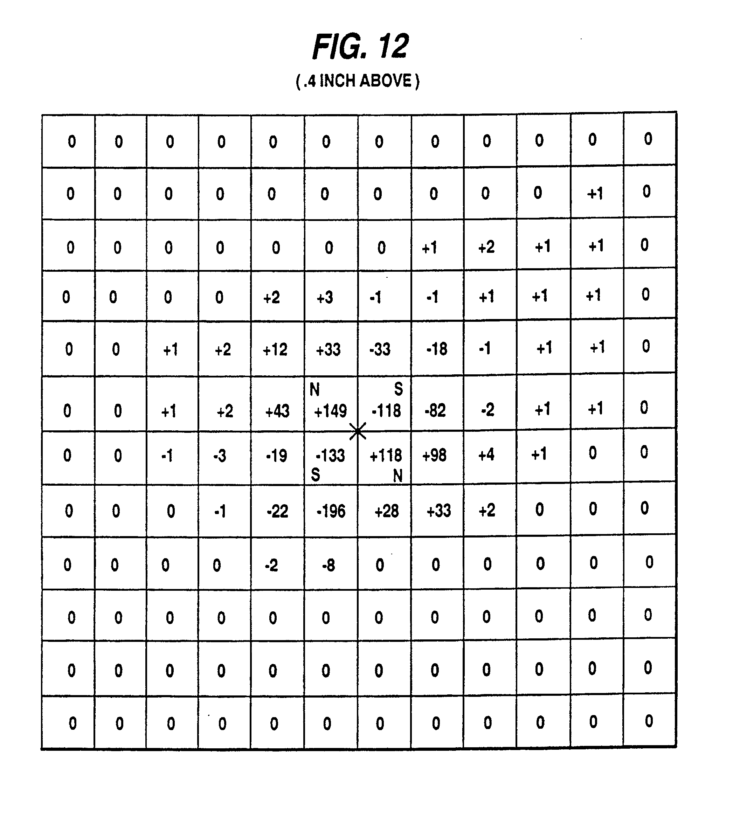

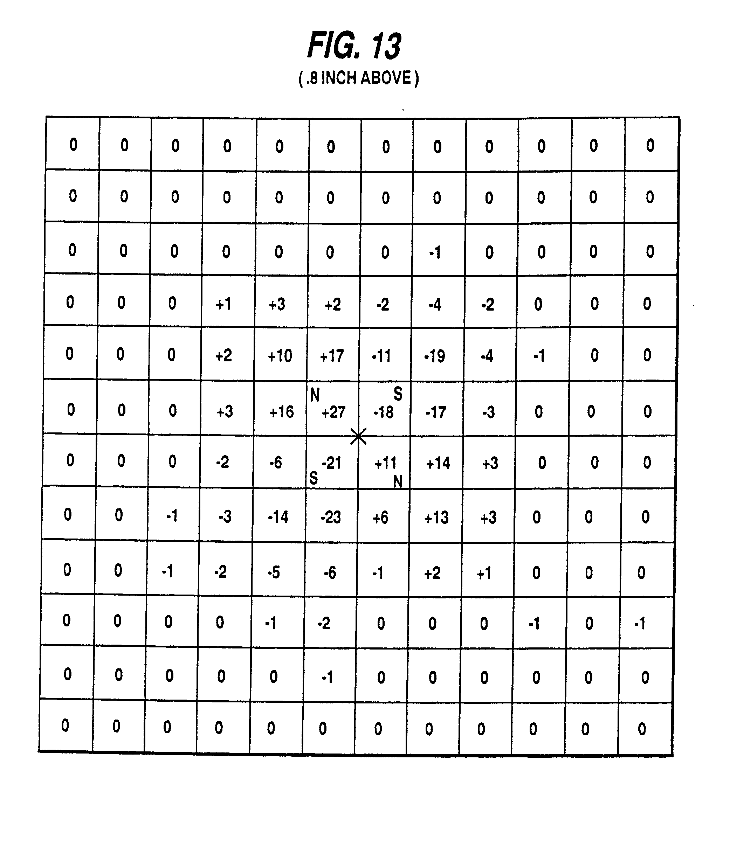

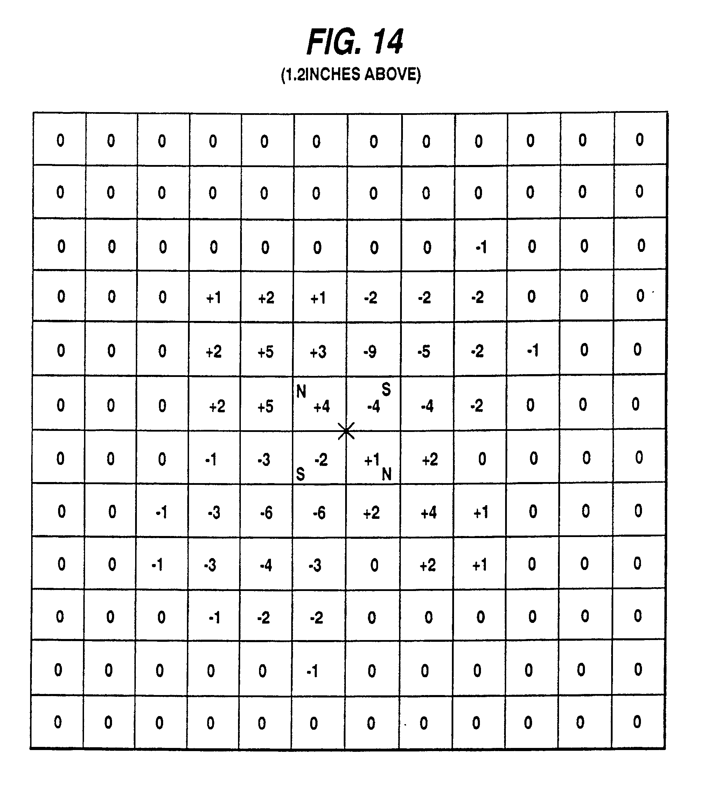

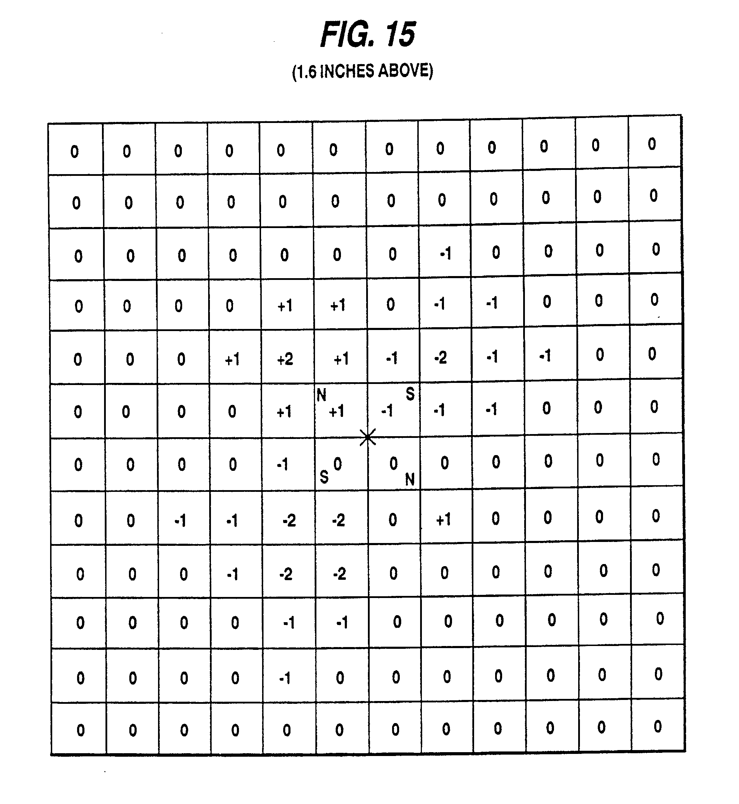

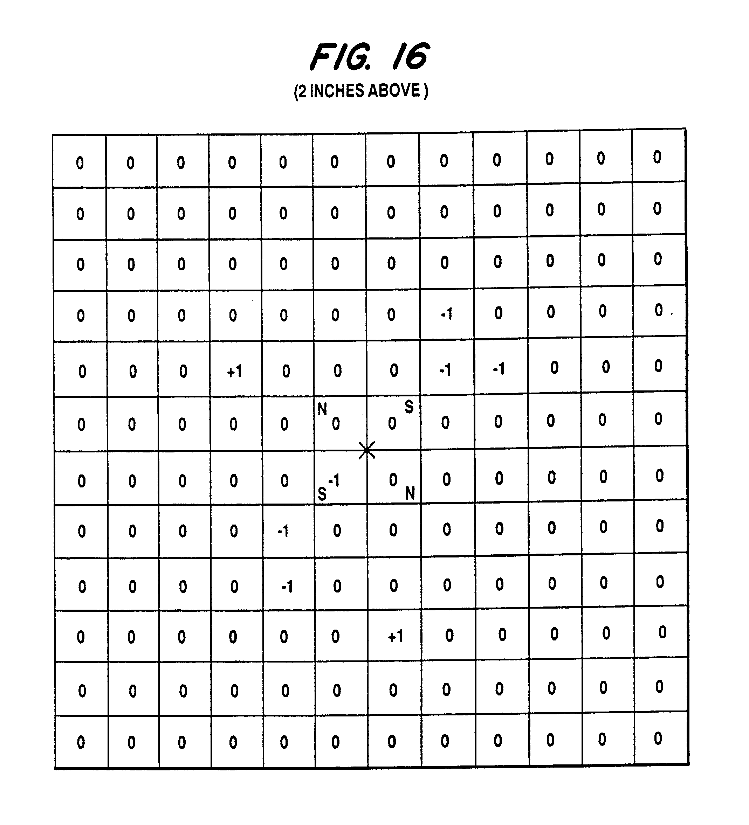

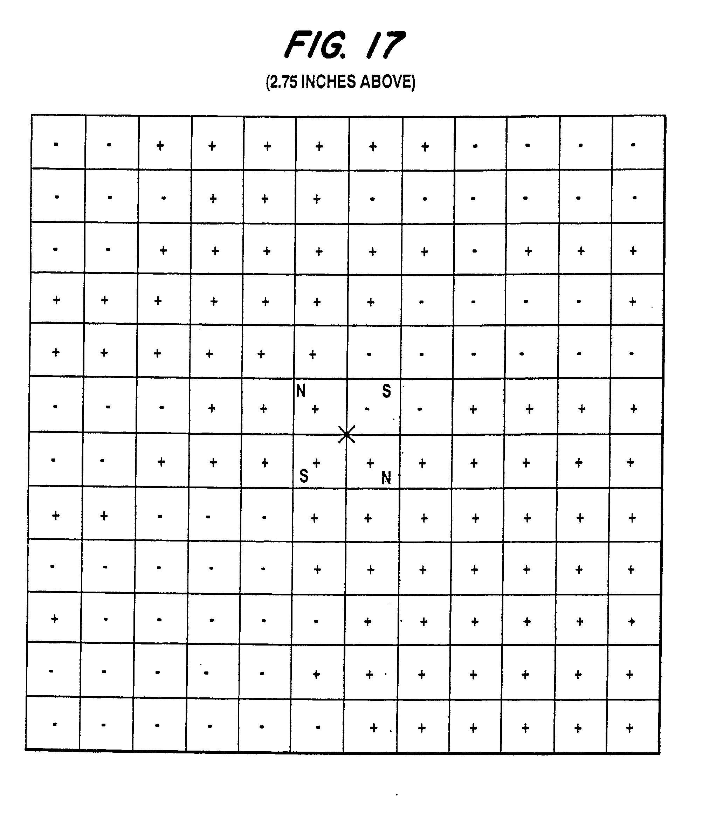

Fig. 11-17 show magnetic field measurements in Gauss taken at various distances from the surface of the magnetic treatment device described with regard to Figs. 9 and 10. The measurements shown in Figs. 11-17 were taken above the magnetic treatment device, and the location of the positive poles of the treatment device are designated by the letter "N" (north) and the negative poles on the face of the treatment device are designated by the letter "S" (south). The measurements were taken at the surface of the magnetic treatment device (Fig. 11), 0.4 inch above the surface (Fig. 12), 0.8 inch above the surface (Fig. 13), 1.2 inches above the surface (Fig. 14), 1.6 inches above the surface (Fig. 15), 2 inches above the surface (Fig. 16), and 2.75 inches above the surface (Fig. 17). The measurement grids of Figs. 11-17 are each 6 inches by 6 inches with individual grid squares of 0.5 inch by 0.5 inch. The Gauss meter was used to measure the magnetic field at the center of each grid square and the measurement was recorded on the corresponding grid square of Figs. 11-17.

As can be seen from this data, measureable magnetic flux extended out two inches from the surface of the treatment device. At 2.75 inches above the surface of the treatment device, the magnetic flux meter used in the test was not sensitive enough to measure specific flux levels, but the measuring instrument was able to distinguish between positive (north seeking pole) and negative (south seeking pole) flux readings as shown in Fig. 17. Thus, it can be seen that a quadripolar magnetic field extends out as far as 2.75 inches from the surface of the magnetic treatment device of the present invention.

Therapeutic magnetic shoes of this invention have been beneficially utilized to increase blood circulation in the feet of persons wearing the shoes. (The increased circulation is brought about by calcium channel blocade in the mouth muscle walls of the blood vessels and capillary beds -- as evidenced by calcium channel blocade in isolated cell preparation.) When blood circulation is increased, the feet maintain an optimum temperature, carry more oxygen to the tissues and thereby bring about a decrease in foot fatigue and post exercise soreness. Demonstrated sodium channel blocade by this device stabilizes nerve cell walls and aids in the reduction of foot pain and discomfort. Tests were conducted on two subjects to evaluate whether the therapeutic magnetic shoe of the invention helped increase blood circulation. Each test subject participated in three test trials. In each trial, the test subject exercised by walking for 30 minutes while wearing exercise shoes. At the conclusion of each exercise period, each subject's shoes and socks were removed and thermograms were taken of the soles of the subject's feet after the feet were allowed to equilibrate to room temperature. A second set of thermograms was taken 15 minutes later. In the first trial, each test subject wore two control exercise shoes having no magnetic treatment devices mounted therein. In the second trial, each test subject wore a control exercise show on the left foot and a therapeutic magnetic shoe according to the invention (as shown in Figs. 1 and 2) on the right foot. In the third trial, each test subject wore therapeutic magnetic shoes according to the invention on both feet.

In both test subjects, comparison of the first and third thermogram sets showed that the soles of each subject's feet were significantly warmer (after the feet were allowed to equilibrate to room temperature) at the end of the third trial (therapeutic magnetic shoes) than at the end of the first trial (control shoes). Similarly, at the end of the second trial, the sole of each subject's right foot (therapeutic magnetic shoe) showed up as being significantly warmer than the sole of the left foot (control shoe). Thus, it was demonstrated that the therapeutic magnetic shoe of the invention maintained foot temperature better than conventional exercise shoes. Such improved temperature regulation is attributable to improved blood circulation in the feet.

Therapeutic magnetic shoes of this invention have been beneficially utilized to control pain of arthritis in the feet of a severe nature, whartons toe and plantar faciaitis.

CASE I

A 56 year old male presented with a history of painful arthritis of both feet. He was unable to walk more than a few blocks without severe pain. He was treated with the therapeutic magnetic shoes for two months after which he was able to walk several miles a day without pain while wearing the therapeutic magnetic shoes of this invention.

CASE II

A 54 year old male presented with arthritis and small blood vessel circulatory compromise. He reported he was unable to walk more than a few yards at a time without intense pain. The pain interferred with his sleep at night. Treatment with the therapeutic magnetic shoes was instituted. After wearing the therapeutic magnetic shoes of this invention for 5 months, he reported he was pain free while wearing the therapeutic magnetic shoes of this invention.

CASE III

A 48 year old male with right Morton's toes and plantar faciaitis of right heel was unable to walk or run without pain. He was treated with the therapeutic magnetic shoes of this invention. He reports he was progressively improved and can walk and run ten miles without pain while wearing the therapeutic magnetic shoes of this invention.

A double-blind double-crossover clinical study is currently being conducted on eighteen test subjects in order to verify the above results.

**Claims**

1. A shoe for providing therapeutic relief to a foot on which the shoe is worn, comprising:
a shoe; and
at least one magnetic treatment device mounted in said shoe and abutting the foot on which said shoe is worn, said magnetic treatment device including a plurality of magnet bodies having at least two positive and two negative magnetic poles substantially in a single plane, said magnetic poles being oriented to define the four vertices of a quadrilateral shape, said two positive poles defining opposite diagonal vertices and said two negative poles defining opposite diagonal vertices of the quadrilateral shape, each of said magnetic poles being magnetically attracted by said two oppositely charged poles and being magnetically repelled by said like charged pole.

2. A therapeutic shoe as claimed in claim 1, wherein said plurality of magnet bodies comprises four magnetic bodies, each having at least one magnetically charged face, two of said magnetic bodies having a positive magnetic pole on one face, and the other two of said magnetic bodies having a negative magnetic pole on said magnetically charged face, said two positive and two negative magnetic poles on said magnetically charged faces of said four magnet bodies being in said quadrilateral orientation.

3. A therapeutic shoe as claimed in claim 2 wherein each of said four magnetic bodies includes two opposite faces, one of said two faces having a positive magnetic pole thereon sand the other of said two faces having a negative magnetic pole thereon.

4. A therapeutic shoe as claimed in claim 3 wherein each of said four magnetic bodies are substantially identical cylinders, each of said magnetic cylinders having two opposite end faces, one of said two end faces having a positive magnetic pole thereon and the other of said two end faces having a negative magnetic pole thereon, the positive magnetic pole end faces of two of said cylinders and the negative magnetic pole end faces of two of said cylinders being in a first plane, and the opposite negative magnetic pole end faces of two of said cylinders and the positive magnetic pole end faces of two of said cylinders being in a second plane.

5. A therapeutic shoe as claimed in claim 4 wherein each of said magnetic cylinders comprises a rare earth magnet.

6. A therapeutic shoe as claimed in claim 5 wherein each of said rare earth magnets comprises a neodymium magnet.

7. A therapeutic shoe as claimed in claim 6 wherein each of said neodymium magnet cylinders has a diameter in the range of 6,35 mm to 12,7 mm and has an energy product of at least 25 MG-Oe.

8. A therapeutic shoe as claimed in claim 4 wherein said quadrilateral shape is a parallelogram shape.

9. A therapeutic shoe as claimed in claim 8 wherein said parallelogram shape is a rectangle shape.

10. A therapeutic shoe as claimed in claim 9 wherein said rectangle shape is a square shape.

11. A therapeutic shoe as claimed in claim 4 wherein said magnetic treatment device further comprises containment means for holding said four magnetic cylinders in said quadrilateral shaped orientation.

12. A therapeutic shoe as claimed in claim 1 wherein said plurality of magnet bodies comprises two elongated magnet bodies, each having a positive magnetic pole at one end and a negative magnetic pole at an opposite end, said two positive magnet poles and said two negative magnetic poles being in said quadrilateral shaped orientation.

13. A shoe for providing therapeutic relief to a foot on which the shoe is worn, comprising:
a shoe having a sole; and
a first magnetic treatment device mounted in the sole of said shoe and abutting the foot on which said shoe is worn, said magnetic treatment device including a plurality of magnet bodies having at least four magnetic poles substantially in a single plane, said magnetic poles being oriented to define the four vertices of a quadrilateral shape, each of said magnetic poles exerting a magnetic force on the other three poles (said two positive poles defining opposite diagonal vertices and said two negative poles defining opposite diagonal vertices of the quadrilateral shape).

14. A therapeutic shoe as claimed in claim 13, further comprising a second magnetic treatment device including a plurality of magnet bodies having at least two positive and two negative magnetic poles substantially in a single plane, said magnetic poles being oriented to define the four vertices of a quadrilateral shape, said two positive poles defining opposite diagonal vertices and said two negative poles defining opposite diagonal vertices of the quadrilateral shape.

15. A therapeutic shoe as claimed in claim 14, wherein said first magnetic treatment device is embedded in said shoe sole in an area abutting a heel of a foot wearing said shoe.

16. A therapeutic shoe as claimed in claim 15 wherein said second magnetic treatment device is embedded in said shoe sole in an area abutting the ball of a foot wearing said shoe.

17. A therapeutic shoe as claimed in claims 13 or 14, wherein said four magnetic poles include two positive and two negative poles, said two positive poles defining opposite diagonal vertices and said two negative poles defining opposite diagonal vertices of the quadrilateral shape, each of said magnetic poles being magnetically attracted by said two oppositely charged poles and being magnetically repelled by said like charged pole.

18. A method of therapeutically increasing circulation and reducing fatigue in a human foot, comprising the steps of:
assembling at least one group of magnet bodies having at least two positive magnetic poles substantially in a single plane and having at least two negative magnetic poles substantially in said single plane;
orienting said two positive poles and said two negative poles of said plurality of magnetic bodies in said single plane to define the four vertices of a rectangle shape with said two positive poles defining opposite vertices and said two negative poles defining opposite diagonal vertices of the rectangle shape;
fixing said orientation of said plurality of magnetic bodies in a first containment body having a flat surface substantially parallel to and proximate said single plane;
fixing said first containment body in a shoe sole having an upper foot abutting surface, said flat surface of said first containment body being fixed in a position substantially coplanar with the upper surface of the shoe sole; and

placing said shoe on the foot of a human in which increased blood circulation, reduced fatigue and reduced pain is desired.

19. A method as claimed in claim 18 wherein said step of fixing said first containment body in the shoe of a sole includes the step of positioning and first containment body in the portion of the shoe sole abutting a heel of a foot wearing the shoe.

20. A method as claimed in claim 19 further comprising the steps of producing a second containment body by repeating said steps of assembling, orienting and fixing a plurality of magnetic bodies in a containment body with a flat surface; and

fixing said flat surface of said second containment body in said shoe sole in a position substantially coplanar with the upper surface of the shoe sole in a portion of the shoe sole abutting the ball of a foot wearing the shoe.

*FIG. 1*

2

5

10

8

4

FIG. 2

# FIG. 3

# FIG. 4

# FIG. 6

# FIG. 5

## FIG. 7

## FIG. 8

## FIG. 9

FIG. 10

GAUSS READINGS
ONE-HALF INCH

# FIG. 11
## ( SURFACE )

| | | | | | | | | | | | |
|---|---|---|---|---|---|---|---|---|---|---|---|
| 0 | 0 | 0 | 0 | 0 | 0 | 0 | 0 | 0 | 0 | 0 | 0 |
| 0 | 0 | 0 | 0 | 0 | 0 | 0 | 0 | 0 | 0 | 0 | 0 |
| 0 | 0 | 0 | 0 | 0 | 0 | 0 | 0 | 0 | 0 | 0 | 0 |
| 0 | 0 | 0 | -1 | -4 | -2 | +10 | +20 | +10 | +10 | 0 | 0 |
| 0 | 0 | -1 | -4 | -10 | +18 | -19 | +23 | +10 | +3 | +2 | 0 |
| 0 | 0 | -1 | -5 | +39 | N +1928 | S -1800 | -36 | +8 | +1 | 0 | 0 |
| 0 | 0 | +1 | +3 | -9 | -1988 S | +1900 N | +61 | -5 | -1 | 0 | 0 |
| 0 | +1 | +2 | +6 | +16 | -20 | -6 | -26 | -8 | -1 | 0 | 0 |
| 0 | +1 | +1 | +3 | +6 | +5 | -4 | -6 | -3 | 0 | 0 | 0 |
| 0 | 0 | 0 | +1 | +1 | 0 | -1 | -2 | -2 | -1 | 0 | 0 |
| 0 | 0 | 0 | 0 | +1 | +1 | -1 | -2 | -1 | -1 | 0 | 0 |
| 0 | 0 | 0 | 0 | 0 | 0 | 0 | 0 | 0 | 0 | 0 | 0 |

## FIG. 12

### ( .4 INCH ABOVE )

| | | | | | | | | | | | |
|---|---|---|---|---|---|---|---|---|---|---|---|
| 0 | 0 | 0 | 0 | 0 | 0 | 0 | 0 | 0 | 0 | 0 | 0 |
| 0 | 0 | 0 | 0 | 0 | 0 | 0 | 0 | 0 | 0 | +1 | 0 |
| 0 | 0 | 0 | 0 | 0 | 0 | 0 | +1 | +2 | +1 | +1 | 0 |
| 0 | 0 | 0 | 0 | +2 | +3 | -1 | -1 | +1 | +1 | +1 | 0 |
| 0 | 0 | +1 | +2 | +12 | +33 | -33 | -18 | -1 | +1 | +1 | 0 |
| 0 | 0 | +1 | +2 | +43 | N +149 | S -118 | -82 | -2 | +1 | +1 | 0 |
| 0 | 0 | -1 | -3 | -19 | -133 S | +118 N | +98 | +4 | +1 | 0 | 0 |
| 0 | 0 | 0 | -1 | -22 | -196 | +28 | +33 | +2 | 0 | 0 | 0 |
| 0 | 0 | 0 | 0 | -2 | -8 | 0 | 0 | 0 | 0 | 0 | 0 |
| 0 | 0 | 0 | 0 | 0 | 0 | 0 | 0 | 0 | 0 | 0 | 0 |
| 0 | 0 | 0 | 0 | 0 | 0 | 0 | 0 | 0 | 0 | 0 | 0 |
| 0 | 0 | 0 | 0 | 0 | 0 | 0 | 0 | 0 | 0 | 0 | 0 |

# FIG. 13
### (.8 INCH ABOVE)

| | | | | | | | | | | | |
|---|---|---|---|---|---|---|---|---|---|---|---|
| 0 | 0 | 0 | 0 | 0 | 0 | 0 | 0 | 0 | 0 | 0 | 0 |
| 0 | 0 | 0 | 0 | 0 | 0 | 0 | 0 | 0 | 0 | 0 | 0 |
| 0 | 0 | 0 | 0 | 0 | 0 | 0 | -1 | 0 | 0 | 0 | 0 |
| 0 | 0 | 0 | +1 | +3 | +2 | -2 | -4 | -2 | 0 | 0 | 0 |
| 0 | 0 | 0 | +2 | +10 | +17 | -11 | -19 | -4 | -1 | 0 | 0 |
| 0 | 0 | 0 | +3 | +16 | N +27 | S -18 | -17 | -3 | 0 | 0 | 0 |
| 0 | 0 | 0 | -2 | -6 | -21 S | +11 N | +14 | +3 | 0 | 0 | 0 |
| 0 | 0 | -1 | -3 | -14 | -23 | +6 | +13 | +3 | 0 | 0 | 0 |
| 0 | 0 | -1 | -2 | -5 | -6 | -1 | +2 | +1 | 0 | 0 | 0 |
| 0 | 0 | 0 | 0 | -1 | -2 | 0 | 0 | 0 | -1 | 0 | -1 |
| 0 | 0 | 0 | 0 | 0 | -1 | 0 | 0 | 0 | 0 | 0 | 0 |
| 0 | 0 | 0 | 0 | 0 | 0 | 0 | 0 | 0 | 0 | 0 | 0 |

# FIG. 14

## (1.2INCHES ABOVE)

| 0 | 0 | 0 | 0 | 0 | 0 | 0 | 0 | 0 | 0 | 0 | 0 |
|---|---|---|---|---|---|---|---|---|---|---|---|
| 0 | 0 | 0 | 0 | 0 | 0 | 0 | 0 | 0 | 0 | 0 | 0 |
| 0 | 0 | 0 | 0 | 0 | 0 | 0 | 0 | -1 | 0 | 0 | 0 |
| 0 | 0 | 0 | +1 | +2 | +1 | -2 | -2 | -2 | 0 | 0 | 0 |
| 0 | 0 | 0 | +2 | +5 | +3 | -9 | -5 | -2 | -1 | 0 | 0 |
| 0 | 0 | 0 | +2 | +5 | N +4 | S -4 | -4 | -2 | 0 | 0 | 0 |
| 0 | 0 | 0 | -1 | -3 | S -2 | +1 N | +2 | 0 | 0 | 0 | 0 |
| 0 | 0 | -1 | -3 | -6 | -6 | +2 | +4 | +1 | 0 | 0 | 0 |
| 0 | 0 | -1 | -3 | -4 | -3 | 0 | +2 | +1 | 0 | 0 | 0 |
| 0 | 0 | 0 | -1 | -2 | -2 | 0 | 0 | 0 | 0 | 0 | 0 |
| 0 | 0 | 0 | 0 | 0 | -1 | 0 | 0 | 0 | 0 | 0 | 0 |
| 0 | 0 | 0 | 0 | 0 | 0 | 0 | 0 | 0 | 0 | 0 | 0 |

# FIG. 15

## (1.6 INCHES ABOVE)

| 0 | 0 | 0 | 0 | 0 | 0 | 0 | 0 | 0 | 0 | 0 | 0 |
|---|---|---|---|---|---|---|---|---|---|---|---|
| 0 | 0 | 0 | 0 | 0 | 0 | 0 | 0 | 0 | 0 | 0 | 0 |
| 0 | 0 | 0 | 0 | 0 | 0 | 0 | -1 | 0 | 0 | 0 | 0 |
| 0 | 0 | 0 | 0 | +1 | +1 | 0 | -1 | -1 | 0 | 0 | 0 |
| 0 | 0 | 0 | +1 | +2 | +1 | -1 | -2 | -1 | -1 | 0 | 0 |
| 0 | 0 | 0 | 0 | +1 | N +1 | -1 S | -1 | -1 | 0 | 0 | 0 |
| 0 | 0 | 0 | 0 | -1 | S 0 | 0 N | 0 | 0 | 0 | 0 | 0 |
| 0 | 0 | -1 | -1 | -2 | -2 | 0 | +1 | 0 | 0 | 0 | 0 |
| 0 | 0 | 0 | -1 | -2 | -2 | 0 | 0 | 0 | 0 | 0 | 0 |
| 0 | 0 | 0 | 0 | -1 | -1 | 0 | 0 | 0 | 0 | 0 | 0 |
| 0 | 0 | 0 | 0 | -1 | 0 | 0 | 0 | 0 | 0 | 0 | 0 |
| 0 | 0 | 0 | 0 | 0 | 0 | 0 | 0 | 0 | 0 | 0 | 0 |

# FIG. 16

## (2 INCHES ABOVE )

| | | | | | | | | | | | |
|---|---|---|---|---|---|---|---|---|---|---|---|
| 0 | 0 | 0 | 0 | 0 | 0 | 0 | 0 | 0 | 0 | 0 | 0 |
| 0 | 0 | 0 | 0 | 0 | 0 | 0 | 0 | 0 | 0 | 0 | 0 |
| 0 | 0 | 0 | 0 | 0 | 0 | 0 | 0 | 0 | 0 | 0 | 0 |
| 0 | 0 | 0 | 0 | 0 | 0 | 0 | -1 | 0 | 0 | 0 | 0 |
| 0 | 0 | 0 | +1 | 0 | 0 | 0 | -1 | -1 | 0 | 0 | 0 |
| 0 | 0 | 0 | 0 | 0 | N 0 | S 0 | 0 | 0 | 0 | 0 | 0 |
| 0 | 0 | 0 | 0 | 0 | S -1 | 0 N | 0 | 0 | 0 | 0 | 0 |
| 0 | 0 | 0 | 0 | -1 | 0 | 0 | 0 | 0 | 0 | 0 | 0 |
| 0 | 0 | 0 | 0 | -1 | 0 | 0 | 0 | 0 | 0 | 0 | 0 |
| 0 | 0 | 0 | 0 | 0 | 0 | +1 | 0 | 0 | 0 | 0 | 0 |
| 0 | 0 | 0 | 0 | 0 | 0 | 0 | 0 | 0 | 0 | 0 | 0 |
| 0 | 0 | 0 | 0 | 0 | 0 | 0 | 0 | 0 | 0 | 0 | 0 |

# FIG. 17

## (2.75 INCHES ABOVE)